# EUROPEAN PATENT SPECIFICATION

(11) **EP 1 333 786 B1**
(45) Date of publication and mention of the grant of the patent: **27.07.2005**
(21) Application number: 00973884.0
(22) Date of filing: 26.10.2000
(51) Int. Cl.: A61F 7/10

(54) **HEAD AND SPINE COOLING DEVICE**
KOPF- UND NACKENKÜHLGERÄT
DISPOSITIF DE REFROIDISSEMENT DE LA TETE ET DE LA COLONNE VERTEBRALE

(43) Date of publication of application: 13.08.2003
(73) Proprietor: Latham, Jeffrey Wade, San Marcos, TX 78666 (US)
(72) Inventor: Latham, Jeffrey Wade, San Marcos, TX 78666 (US)
(74) Representative: Wardle, Callum Tarn
(86) International application number: PCT/US2000/029509
(87) International publication number: WO 2002/034177

(56) References cited:
- WO-A-94/00087
- WO-A-99/08632
- DE-A- 2 948 059
- US-A- 4 138 743
- US-A- 5 897 581
- US-B1- 6 183 501

## Description

### Technical Field of the Invention

The present invention relates, in general, to the field of head, neck, and spine medical treatment, and more particularly, to a cooling device for the brain and spinal cord of an individual with a brain or spinal cord injury for reducing trauma to those areas.

### Background of the Invention

Without limiting the scope of the invention, its background is described in connection with brain and spinal cord treatment, as an example.

The brain and spinal cord form the central nervous system (CNS), the body's chief controlling and coordinating centers. The brain, which is housed in the skull, is the major organ of the body for control of all the body's voluntary and involuntary activities. The principal parts of the brain are the brain stem, the diencephalon, the cerebrum, and the cerebellum. Cranial bones and the cranial meninges protect the brain and cerebrospinal fluid serves as a shock absorber for the brain and circulates nutritive substances from the blood to the brain. A large round hole called the foramen magnum is located at the bottom of the skull. It is through the foramen magnum that the spinal cord passes down from the brain into the spine. The spine is a bony column which serves as a protective surrounding for the spinal cord.

When an area of the body collides with an external source in its surroundings, severe trauma and swelling of the tissue may occur in the injured area. To reduce swelling, treatment often consists of lowering the temperature of the injured area.

Lowering temperature is often achieved by applying a cold element or substance to the injured area. In some instances, the treatment has been as simple as applying ice to the location of the injury. More sophisticated methods have included applying cold packs to the injury. Heretofore, in this field, cold therapy has generally been limited to the limbs of the body including the leg (particularly the knee), the arm, and the shoulder. Treatment of this type has generally been applied most consistently in situations involving athletic injuries. Cold therapy has also been used for aesthetic purposes such as applying cold packs to the face to reduce bags under the eyes and for purposes of reducing the pain of headaches. Therapy has generally required refrigeration of the packs.

WO-A-9 908 632 discloses a cooling system according to the preamble of claim 1.

When the brain or spinal cord is traumatized due to injury, the extent of the trauma to the brain or spinal cord is not always readily apparent. The collision of an individual's head with external surroundings causes the brain to collide with the individual's skull, which may produce swelling of the brain. Swelling can restrict the flow of fluids that normally circulate around the brain and, potentially, cause the fluids to accumulate and therefore compress the brain down into the floor of the skull and cervical bone of the spine. The collision of the individual's head or body with external surroundings may cause injury to the spinal cord and result in swelling. To reduce the effects of this secondary trauma, the present invention can be placed over the head, neck, and spinal regions to lower the temperature in those areas and help reduce swelling. The reduced swelling of the brain reduces the potential for more serious injury to the individual.

Therefore, there is a need for a device that may be easily transported and applied in emergency situations but may also be used in rehabilitative environments. Also, there is a need for a device that requires no special storage conditions such that implementation of such a device requires extensive redesign of, or requires, additional space for storage facilities. Furthermore, there is a need for a device that may be flexibly and easily adapted to an individual at the scene of an accident while not adding additional stress or pressure on the individual.

### Summary of the Invention

The present invention disclosed herein is a cooling system as defined in the appended set of claims. It includes a head and neck device that can be cooled to reduce trauma to the brain. The cooling device facilitates cooling of the head and neck that reduces swelling of the brain. Reduction of swelling of the brain helps to decrease both short and long term damage to the brain of a patient.

In one embodiment, the cooling system includes a head device and a neck brace. The head device has a top panel and a back panel; each panel capable of housing a cold element to facilitate cooling. The top panel may include one or more fastening devices to secure the head device to the head of the individual. The front panel may also include one or more orifices to facilitate access to the ears of the individual. The back panel of the head piece may include one or more fastening devices and a bottom appendage; the fastening device being positioned to come into contact with the one or more fastening devices located on the front panel for complete coverage of the head and over the carotid arteries. The back panel may be elongated by attaching a back panel strip that covers the back along the spinal cord for cooling.

The cooling system may also include a neck brace having front and back brace members and a chin support. The neck brace may further include one or more orifices that allow air to circulate to reduce heat buildup which may cause sweating and discomfort of the individual. The neck brace may also have a hole for, e.g., facilitating a tracheotomy to assist the individual in breathing, if necessary. The neck brace may also include a fastening device to secure the neck brace to the individual and to support the head device.

In another embodiment, the cooling system of the present invention may be a one-piece head device. The head device includes an opening for facial exposure and is capable of housing a cooling element. The head device further includes a flexible material.

A further embodiment provides a cooling system having a top panel with one or more cooperating fasteners and a right side and a left side corresponding to the right side and the left side, respectively, of the individual. A first elongate neck member extends from the right side and a second elongate neck member extends from the left side so the neck members are disposed substantially over the carotid arteries of the neck when worn by said individual. The system further provides activatable cooling medium housed in the top panel and the neck members so the cooling medium cools the head and cools blood flowing to the head from the carotid arteries of the neck upon activation of the cooling medium. The system may be adapted for use with recreational, sports or occupational head protection.

A still further embodiment provides a system for cooling blood flowing to the brain of an individual from the neck by means of a flexible neck member housing activatable cooling medium. The flexible neck member includes a body and first and second members extending laterally from the body. The first and second members further have cooperating perimeter contours. A spine support portion extends from the body between said first and second members. Fasteners are provided on the first and second members. The first and second members wrap around the neck of the individual so that the first and second members are fastenable together by the fasteners and so that the cooperating perimeter contours of the first and second members cooperate to form an orifice that allows access to the front of the neck of the individual. Activation of the coolant medium cools blood flowing to the brain from the neck.

In yet another embodiment, the cooling system of the present invention comprises a cooling device and a cooling element system. The cooling element system includes a connecting mechanism whereby the cooling element may enter the cooling device, a storage device for housing said cooling element, and a release system.

The present invention allows Emergency Medical Services (EMS) personnel to monitor bleeding of the individual, if any. The present invention also facilitates management of the airway by allowing for tracheotomy treatment. Because of the facial opening of the present invention, the eyes, nose and mouth of the individual may be monitored.

### Brief Description of the Drawings

For a more complete understanding of the features and advantages of the present invention, reference is now made to the detailed description of the invention along with the accompanying figures in which corresponding numerals in the different figures refer to corresponding parts and in which:
Figure 1 is a cross sectional view of the brain with cerebrospinal fluid;
Figure la is a cross sectional view of the brain with cerebrospinal fluid after a traumatic injury;
Figure 2 is a schematic illustration of one embodiment of the present invention as placed on an accident individual portraying the left side view;
Figure 3 is a schematic illustration of the left side view of one embodiment of the present invention in application;
Figure 4 is a schematic illustration of the top view of the top panel of the head device of one embodiment of the present invention;
Figure 5 is a schematic illustration of the top view of the back panel of the head device of one embodiment of the present invention;
Figure 5a is a frontal view of a back panel strip for cooling the spinal column of one embodiment of the present invention;
Figure 6 is a schematic illustration of another embodiment of the present invention as placed on a medical patient portraying the side view; and
Figure 7 is a frontal view of yet another embodiment of the present invention having a cooling element system fluidly connected to the head device.
Figure 8a is a back view of an alternative embodiment of the present invention.
Figure 8b is a side view of the embodiment of Fig. 8a shown being worn by an individual.

### Detailed Description of the Invention

The present invention disclosed herein includes a head device and a cooling medium for cooling the head, neck and spine. Cooling of the head, neck and spine facilitates a reduction in swelling of the brain and surrounding tissue after injury, which helps to minimize long term damage to the brain.

It has been reported that approximately 20 percent of all head injuries are classified as severe and hence may be potentially life threatening. The overall effects of traumatic brain injuries are permanent and complex, thus, management of traumatic brain injury is of critical importance. Management of the effects of the injuries during the critical time period following severe brain injury or during what professionals in emergency medicine have termed the golden hour affects the survival and recovery of those individuals. This golden hour includes the time the patient is at the scene of the accident and the essential field stabilization and transport of the individual to a medical facility. The golden hour also includes a window of opportunity to determine how extensive the damage is to the brain. Neurologists may need to perform a CT scan to evaluate the extent of damage to the brain and to determine if there are bleeds, lesions contusions to the brain, and/or fractures to the skull. On-scene intervention also includes procedures that are as unobtrusive as possible and limit damage to the brain. Current emergency protocols require checking airway, breathing, and circulation (ABC's)followed by neck and spinal stabilization when neck and back injury appears to be evident before transport.

The present invention is useful not only for emergency response personnel, but also for individuals who engage in sports or recreation where head protection or head cooling may be desirable. The present invention may be incorporated into helmets for bicycle and motorcycle riding, car racing, boxing, baseball, skate boarding, football, hockey, climbing, skydiving and other sport and recreational helmets. Other uses may be found in occupational helmets such as construction helmets, miner helmets and other head protection for work or dangerous environments. The cooling function of the present invention may be self activated by the user to cool the head for comfort or in the case of injury before emergency response personnel arrive.

### Structure

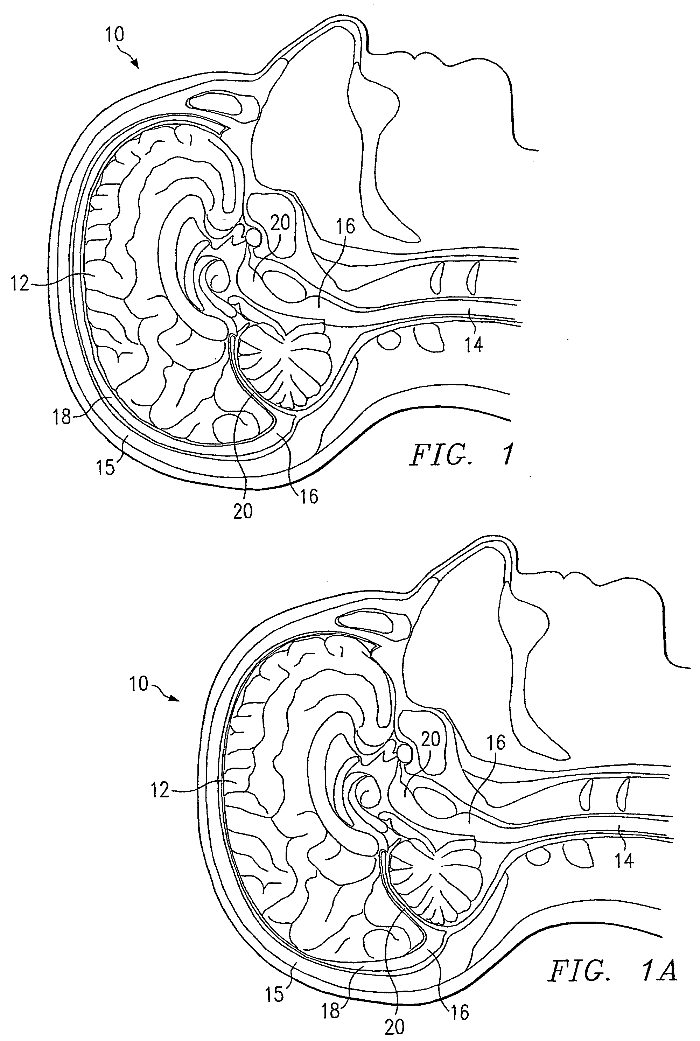

Figure 1 is a cross sectional view of the brain with cerebrospinal fluid. The brain 12, as well as the spinal cord 14, is generally protected against injury by cerebrospinal fluid 16. The cerebrospinal fluid 16 circulates through the subarachnoid space 18 that is around the brain 12 and spinal cord 14. The cerebrospinal fluid 16 also circulates through the brain 12 via ventricles 20. Normally, cerebrospinal fluid 16 is absorbed as rapidly as it is formed. Cerebrospinal fluid generally leaves the body at the same rate it is produced, about 0.47 milliliters per hour, through the arachnoid space 18 at the top of the skull 15. The cerebrospinal fluid 16 is a clear, colorless, fluid having a watery consistency and contains vital nutrients including proteins, glucose, salts, and white blood cells. The cerebrospinal fluid 16 also circulates nutrients delivered via the blood.

Figure 1a is a cross sectional view of a brain having swelled against the skull 15. When a brain injury occurs there is primary physical damage that can kill or disable. But there are also secondary insults to the brain 12, which result from the swelling of the brain 12. When swelling occurs, the brain 12 will swell against the skull 15, thus blocking the flow of the cerebrospinal fluid 16 around the brain 12 and blocking the flow of nutrients through the ventricles 20. The internal pressure builds and squeezes blood vessels blocking the flow of blood which carries oxygen and nutrients to the brain. A reduction in the flow of nutrients to the brain 12 also causes secondary damage to the brain 12.

The build up of fluids 16 causes inter-cranial pressure (ICP) to build and the brain swells against the skull and blocks the exit point of cerebrospinal fluid 16. Pressing against the wall of the skull also damages the brain. All of the above cause additional or secondary injury to the brain. The elevation of the heart rate increases more blood to the injured area of the brain, thereby causing more damage due to swelling and the presence of blood destroying brain cells.

The present inventor has recognized that application of a cold element to the head, neck, and/or spine reduces the swelling of the brain and spinal cord after an injury and reduces the increased flow of blood by means of cooling the carotid artery. Reducing swelling is particularly important in emergency situations such as vehicle accidents which involve head and/or spinal cord injuries as there is eminent potential for build up of blood and cerebrospinal fluid in the cranial cavity. Damage is further intensified because the heart rate is increased as a natural response to trauma, thereby increasing profusion pressure (more blood to the injured site). In the case of penetrating or open head injuries, the problem of increased pressure due to blockage or exit capabilities is naturally reduced as the skull has been penetrated, but reducing the flow of blood to the area may help to reduce the amount of hemorrhaging.

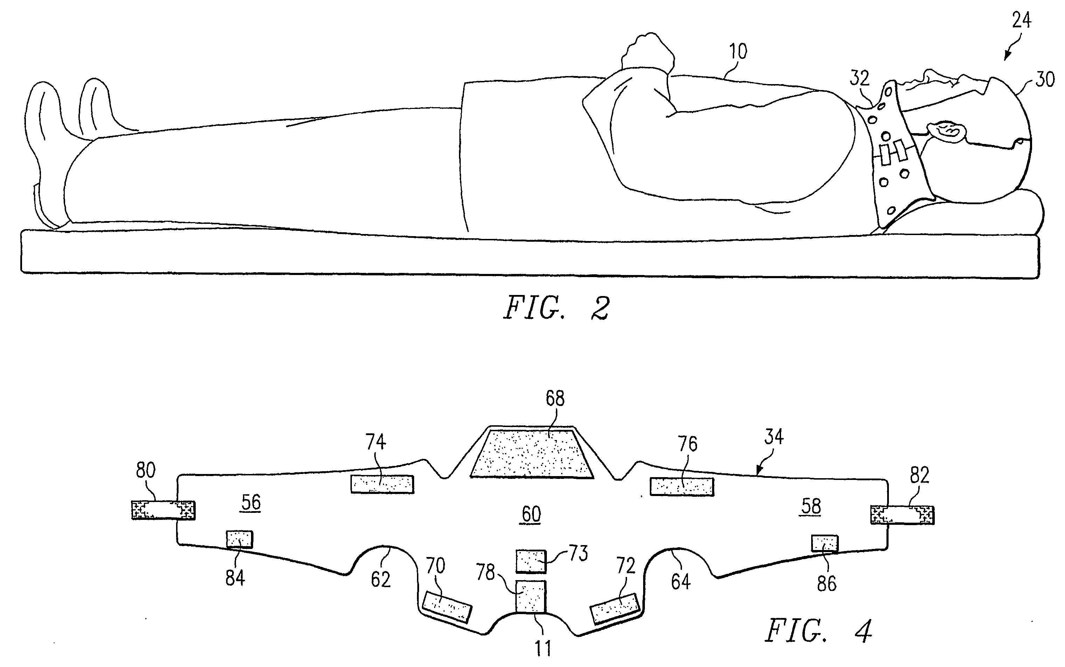

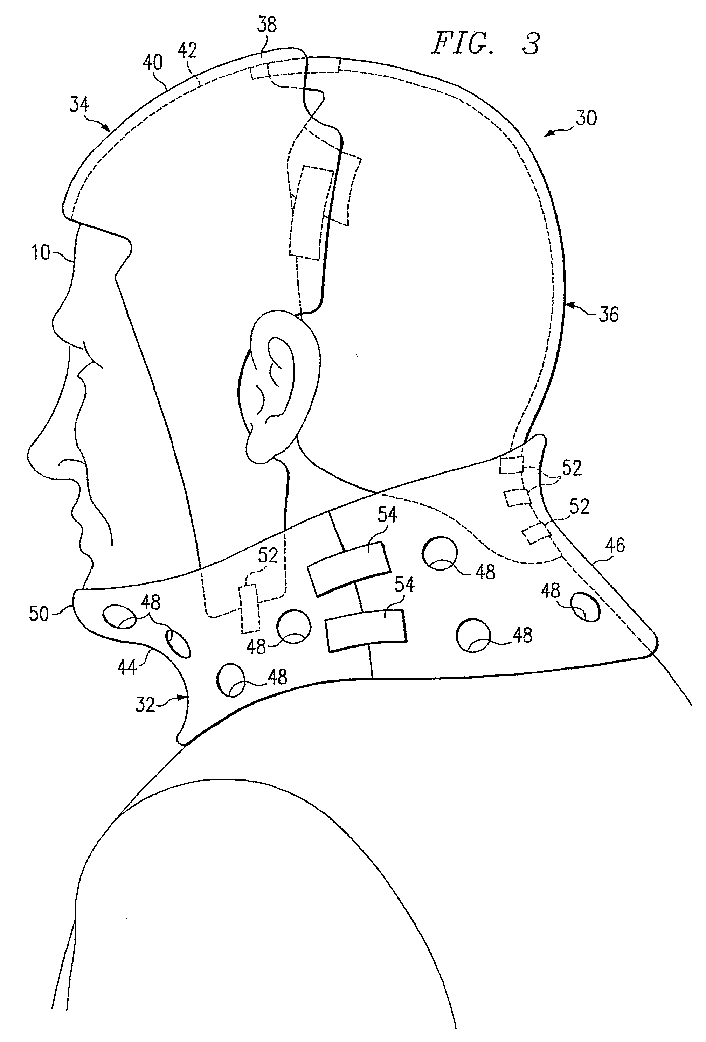

Figure 2 is a schematic illustration of one embodiment of the present invention as placed on an accident individual. The accident individual 10 is found immobile at the scene of the accident. Emergency Medical Services (EMS) has standard guidelines by which treatment is administered to brain and spinal cord injury individuals. EMS will perform the ABC's and make an assessment using the Glascow Coma Scale (GCS) which assesses the person's level of consciousness by eye opening, visual tracking, response to pain, and environmental stimulation to determine if a brain injury is suspected. If the person is unconscious, the stabilization of the head, neck, and back is completed. EMS personnel will be able to place the cooling system 24 on the head of the individual 10 in order to reduce intercranial pressure consequently reducing trauma to the brain and in turn, reducing or minimizing brain damage. The cooling system 24 is mounted on the head and neck of individual 10 through the use of head device 30 and neck brace 32. The cooling system 24 stabilizes and cools the head and neck of individual 10. The use of the cooling system 24 can be used in addition to current neurological protocols as an effort to reduce the need for a shunt, thereby reducing the need for invasive procedures to an already injured site.

Referring to Figure 3, the head device 30 includes a top panel 34, a back panel 36, and a back panel strip 37 all of which are made of flexible material 38. Flexible material 38 may be, e.g., plastic, flexible foam, cloth, paper, or rubber. The flexible material 38 may have non-metallic properties that allow the head device 30 to be worn into a magnetic resonance imaging ("MRI")machine after individual 10 is brought to a medical facility. Head device 30 also includes a top layer 40 and bottom layer 42 that allow the cooling properties of cooling system 24 to be activated.

The neck brace 32 may include a front brace member 44, a back brace member 46, and one or more orifices 48 for air circulation. Neck brace 32 may also include a chin support 50 that permits the neck brace 32 to be more comfortably fitted to individual 10. The brace members 44 and 46 may include one or more inside fastening devices 52 and one or more outside fastening devices 54, that permit the brace members 44 and 46 to be fastened and permit the head device 30 to be interfaced to the neck brace 32 and provide a rigid support for the head of individual 10.

Referring to Figure 4, the top panel 34 having a substantially rectangular shape has a first elongated member 56, a second elongated member 58, and a central portion 60. The elongated members 56 and 58 extend from the central portion 60 of the top panel 34, which permit the central portion 60 to be positioned on top of the head of individual 10. The elongated members 56 and 58 are generally flush with the sides of the face of individual 10. Elongated member 56 and 58 extend down to facilitate cooling of the carotid arteries. The first and second elongated members 56 and 58 include a first recess 62 and a second recess 64, respectively, to provide openings for the ears of individual 10.

Accessibility to the ears of individual 10 allows EMS to provide medical treatment, if necessary, as bleeding from the ears can result from traumatic brain injuries. Accessibility to the ears of individual 10 also allows the patient to hear EMS personnel. The central portion 60 may include a third recess 71 for the hair of individual 10. The top panel 34 may further include a fastening device 68 (located on central portion 60), a left panel fastening device 70 and right panel fastening device 72 (also located on central portion 60). Left panel fastening device 70 is generally located below and adjacent fastening device 68 and is generally parallel to right panel fastening device 72, which is located below and to the right of the fastening device 68. The fastening devices 68, 70, and 72 are positioned to moveably connect to a fastening strap 73 that secures the head device 30 to individual 10.

The top panel 34 may also include a first and second reflective member 74 and 76, respectively, whereby the head of individual 10 is readily visible in dark and low visibility conditions. The top panel 34 may also include a bottom fastening device 78 located on central portion 60 that permits the back panel 36 to be connected and provides full coverage of the head of individual 10. The top panel 34 may also include left and right end tabs 80 and 82, located at the ends of elongated members 56 and 58 ,respectively. Located adjacent of end tabs 80 and 82 are end fastening devices 84 and 86.

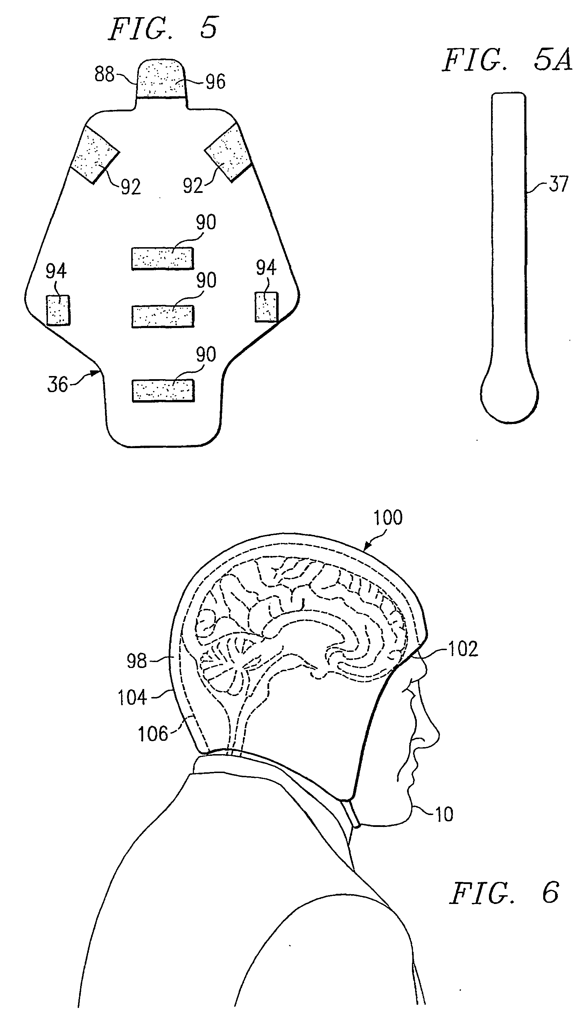

Now referring to Figure 5, the back panel 36 is depicted having an appendage 88, a first one or more fastening devices 90, a second one or more fastening devices 92, and a third one or more fastening devices 94. The one or more first fastening devices 90 are generally positioned in parallel on back panel 36 and used to connect the back panel 36 to the neck brace 32. The second one or more fastening devices 92 are fixedly attached to the back panel 36 and connect the sides of back panel 36 to the elongated members 54 and 56 of the front panel 34. The appendage 88 has an appendage fastening device 96 fixedly attached to the appendage 88. The appendage fastening device 96 of appendage 88 and the third one or more fastening devices 94 attach to the cooling strap 73 to secure the head device 30 to the head of individual 10. A back panel strip 37 (Fig. 5a) may be attached to the back panel 36 to facilitate cooling of the spinal cord area.

The cooling medium 98 may be any substance that provides cooling properties to the head device. The cooling medium may include chemical packets that are activated by application of pressure to the packet resulting in an endothermic reaction. The cooling medium may also include ice or generic ice packs that are refrigerated. The cooling medium generally will lower the temperature of the head approximately one to approximately two degrees and may include a large number of packets or changing of packets at predetermined intervals such that the head is cooled.

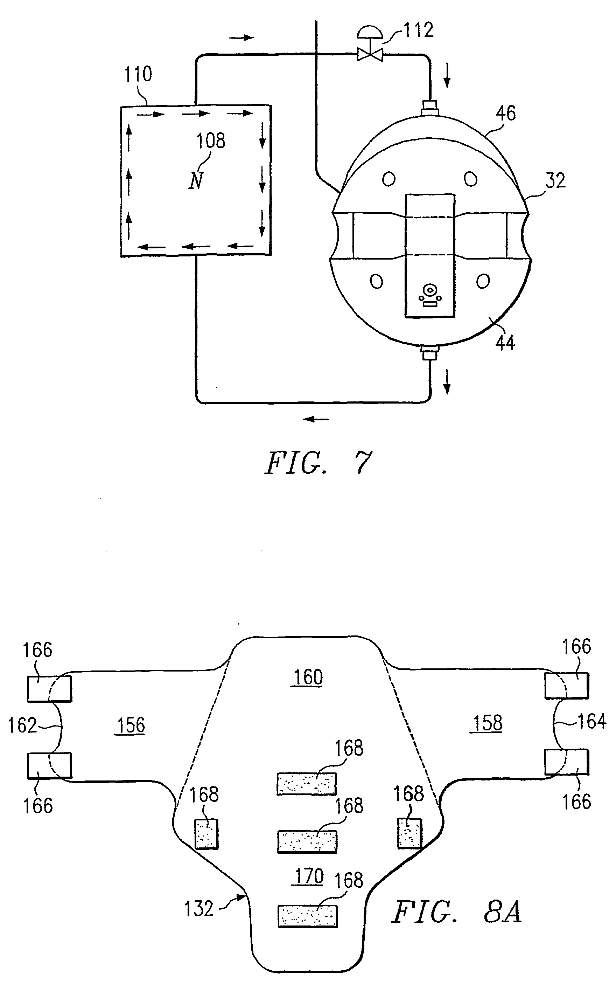

Figure 6, depicts another embodiment of the present invention. The cooling system has a head piece 100 having an opening 102 and a cooling medium 98 which allows the face to be exposed for sight and breathing by the individual 10. The head piece 100 has a top layer 104 and bottom layer 106 and the cooling medium 98. The head piece 100 is suitable for non-emergency situations athletic events, such as boxing, where repeated blows to the head occur. The head piece 100 may also be worn when needed by traumatic brain injury patients that are in rehabilitation therapy when needed. Trauma to the brain requires extensive rehabilitation, which may lead to swelling from time to time and which causes setbacks in recovery. Patients in rehabilitation will generally feel a heating sensation. Such swelling and sensation may be reduced by application of the head piece 100.

Figure 7 is cross-sectional view of yet another embodiment of the present invention. The neck brace 32 and cooling medium system 108 function as integral parts of the neck brace 32. Circulated through the front brace member 44 and the back brace member 46 are a chemical or chemical mixtures that act as coolants. The chemical or chemical mixtures are contained in a tank 110, and valves 112 that are interconnected to the front brace member 44 and the back brace member 46 regulate their circulation. The flow of chemicals allows for immediate reduction of swelling of the brain and may be, e.g. constant, intermittent or under the control of a temperature gauge or feed back system.

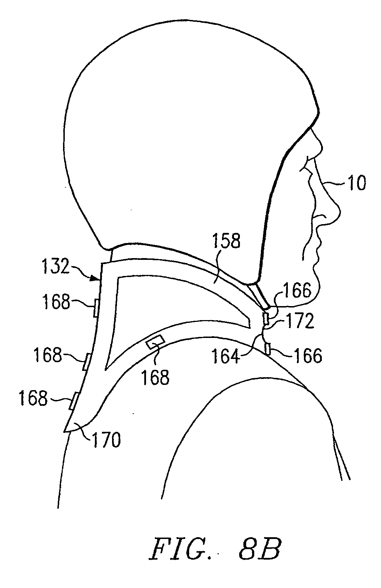

Figures 8a and 8b depict an alternative embodiment of the present invention. In the embodiment of Figs 8a and 8b, wrap-around flexible neck member 132 accomplishes cooling of the neck, and in particular cooling of blood flowing to the head from the carotid arteries of the neck. In Fig. 8a, neck member 132 houses activatable coolant as already described herein and comprises flexible flaps 156 and 158 extending from body 160 of neck member 132. Flaps 156 and 158 comprise cooperating perimeter contours 162 and 164 and cooperating fasteners 166. Additional fasteners 168 may be provided on body 160 of neck member 132 to provide means for attaching neck member 132 to optional components of the embodiment such as a back panel (previously described). Body 160 of neck member 132 further comprises spine support portion 170 extending from body 160.

Fig. 8b depicts the embodiment of Fig. 8a in operation as worn by an individual. Neck member 132 is placed on the back of the neck of individual 10 so that the back face of neck member 132, depicted in Fig. 8a, is distal to the neck of individual 10 and so that spine support portion 170 extends between the shoulder blades of individual 10. Flaps 156 and 158 are wrapped around the front of the neck of individual 10 and fastened together with fasteners 166 so that cooperating contours 162, 164 meet to form orifice 172 to provide access to the front of the neck of individual 10 for tracheotomy or other emergency procedures requiring access to the neck without compromising blood cooling. Neck member 132 houses coolant that may be optionally activated by individual 10 or by someone else in the event individual 10 is incapacitated. Optionally, neck member 10 may be provided with a port through which coolant may be circulated from an exterior source or to provide motive force to circulate coolant already housed in neck member 132. Further optionally, neck member 10 may be provided with an optionally inflatable lining that may be inflated to provide a secure fit around the individual.

The embodiment of Figs 8a and 8b provides a low cost, highly portable embodiment of the invention while preserving the function of cooling blood flowing to the brain from the carotid arteries of the neck to diminish swelling of the brain in the event of head trauma. The present embodiment further provides support for the neck and spine to minimize traumatic movement that might exacerbate the trauma.

### Operation

In operation, if cooling medium 98 is resident in head device 30, such as a single-use or replaceable package, then cooling medium 98 is simply activated and the device 30 is placed on the patient. If the cooling medium 98 is stored outside the head device 30, the cooling medium 98 is activated and then placed within the head device 30. The cooling system 24 is then mounted on the head of individual 10.

The cooling system 24 is mounted by first placing the back panel 36 flat into the inside of back brace member 46 followed by aligning the first one or more fastening devices 90 of back panel 36 with the inside fastening devices 52 of back brace member 46. Fastening devices 90 and 52 are then connected. The top layer 40 of back panel 36 should face the inside of back brace member 46 such that the third one or more fastening devices 94 of back panel 36 are unencumbered by the back brace member 46. The combined back panel 36 and back brace member 46 are then carefully and strategically positioned to the back of the head of individual 10.

Next, the top panel 34 of head device 30 is placed on the top portion of the head of individual 10 with the top fastening device 68 of front panel 34 facing the face of individual 10. Once front panel 34 has been positioned so as to cover the top of individual's 10 head, elongated members 56 and 58 are brought down and around the individual's 10 face to the right and left respectively. As the two elongated members 56 and 58 are lowered, a first recess 62 and a second recess 64 are comfortably positioned around the ears of individual 10 within the recesses 62 and 64. The fastening device 68 of front panel 34 sits in close proximity to the eyes but generally do not cover them. The fastening device 78 of top panel 34 is attached to the fastening device 96 of back panel 36, providing full coverage of the head. The reflective members 74 and 76 should generally be readily visible at each side of the elongated members 56 and 58. The two elongated members 56 and 58 will generally meet at the bottom of individual's 10 chin. The left-end fastening device 80 and the right- end fastening device 82 allow, e.g., EMS, to pull gently to snugly fit to head of individual 10.

The front brace member 44 is placed around the front of individual's 10 neck and the elongated members 56 and 58 such that the chin of individual 10 sits comfortably in the chin support 50 of front brace member 44. The front and back brace members 44 and 46 will generally meet such that the one or more fastening devices are connected to secure the entire cooling system. The cooling strap 73 is then applied centeredly to the front fastening device 96 of top panel 34. Finally, the cooling strap is brought around to attach to the second one or more fastening devices 92 of top panel 34.

In addition to the foregoing embodiments, further embodiments of the invention are contemplated. One such embodiment provides top panel 34 with neck extensions 56, 58 and optionally other elements of the invention described herein above so that cooling is provided to the neck and particularly to blood flowing to the brain from the neck without requiring fitting a helmet over the user's head.

Neck cooling elements 56, 58 or 156, 158 and optionally other components of the invention may be incorporated into sports, recreational or occupational head protection whereby the cooling function may be optionally activated by the wearer for comfort, recovery from fatigue and/or heat, or in the case of emergency such as injury before emergency response personnel arrive. The cooling function may further be activated by a bystander in the event the wearer is incapacitated.

The present invention may further comprise one or more monitors to monitor important biological parameters of the individual such temperature, blood pressure, pulse rate and the like. Such a monitor may comprise one or more sensors in contact with the individual, or having remote sensing capabilities, to detect the desired biological parameter, and one or more displays to display the sensor readings. The monitor may be integral with the various embodiments of the invention described herein, such as sewn into the flexible material of the panels, or the monitor may be permanently or detachably attached or fastened to a panel or other element of the invention. Such detachable fastening may be accomplished with VELCRO fasteners, buckle and strap fasteners, ties, snaps or by any suitable means.

The invention contemplates all modes of implementing the cooling function, including water, ice water, air, chemical coolants or refrigerants, electrical or mechanical cooling and so forth. Cooling may be accomplished by circulating coolant through the elements of the present cooling system, wherein the elements may comprise interconnectable channels for circulating the coolant medium through and among the elements of the system, or by activating stationary coolant housed in the elements of the present system.

Cooling may be activated by various means, including initiating an endothermic chemical reaction by, for example, bringing together previously isolated chemical components of a chemical coolant medium, and/or by opening a valve or port to introduce coolant into the system or to provide a motive force to circulate coolant already housed in the present cooling system.

The panels and braces of the invention may incorporate inflatable members that may be inflated to provide a snug and secure fit of the various elements around the wearer. The inflatable members may be inflated with a suitable fluid including air, water or even the coolant medium itself. The inflatable members may be inflated automatically by, for instance, pulling a tab or pin to release pressurized fluid into the inflatable member. Additionally or alternatively, the inflatable members may be inflated in a regulated manner by, for example, a fluid pressure regulator having a regulatable valve and optionally a pressure gauge, whereby the inflatable member may be regulatably inflated to a desired pressure.

Further embodiments include adapting the invention for use with animals, children and infants.

The foregoing description has been directed to particular embodiments of the invention for the purposes of illustration and explanation. It will be apparent that many alternatives, modifications, and variations will be apparent to those skilled in the art in light of the foregoing disclosure. In particular changes may be made to the shape and size of the head piece and neck brace to accommodate all patients, including humans and animals.

## Claims

1. A cooling system for cooling the head and neck of an individual wearing the system, the system comprising:
a top panel (34) having one or more cooperating fasteners and having a right side and a left side corresponding to the right side and the left side, respectively, of said individual;
a back panel (36) having one or more cooperating fasteners, whereby said top panel is adjustably fastenable to said back panel; and
activatable cooling medium (98) housed in said top panel and said back panel wherein said cooling medium cools the head and cools blood flowing to the head from the carotid arteries of the neck upon activation of said cooling medium,
**characterized by** said top panel further having a first elongate neck member (56) at said right side and a second elongate neck member (58) at said left side whereby said neck members are disposed substantially over the carotid arteries of the neck when worn by said individual.

2. The cooling system as recited in claim 1 wherein said top and back panels comprise a flexible material that conforms to the head and neck of an individual.

3. The cooling system as recited in claim 1 wherein said elongate neck members each further comprise a perimeter contour shaped to provide access to the ears of said individual.

4. The cooling system as recited in claim 1, further comprising a neck brace (32), wherein said top panel having one or more fasteners is moveably fastenable to said neck brace.

5. The cooling system as recited in claim 4, wherein said back panel further comprises a fastener whereby said back panel is moveably fastenable to said neck brace.

6. The cooling system as recited in claim 1, wherein said system comprises a third member housing activable cooling medium whereby said third member is moveably attached to said back panel to provide cooling of the spine upon activation of said cooling medium.

7. The cooling system as recited in claim 4, wherein said neck brace further comprises a flexibly rigid material for support of the neck.

8. The cooling system as recited in claim 4, wherein said neck brace further comprises one or more openings (48) for air ventilation and breathing assistance.

9. The cooling system as recited in claim 4, wherein said neck brace further comprises a front brace member (44) and a back brace member (46).

10. The cooling system as recited in claim 4, wherein said neck brace is further defined as comprising at least one orifice allowing access to the neck of said individual.

## Patentansprüche

1. Kühlsystem zum Kühlen des Kopfes und Halses eines Individuums, das das System trägt, wobei das System umfasst:
eine obere Auflage (34), die ein oder mehrere zusammenwirkende Befestigungselemente aufweist und eine rechte und eine linke Seite aufweist, die der rechten bzw. der linken Seite des Individuums entsprechen;
eine hintere Auflage (36), die ein oder mehrere zusammenwirkende Befestigungselemente aufweist, wobei die obere Auflage an der hinteren Auflage einstellbar befestigbar ist; und
aktivierbares Kühlmittel (98), das in der oberen Auflage und der hinteren Auflage benutzt wird, wobei bei Aktivierung des Kühlmittels das Kühlmittel den Kopf kühlt und Blut kühlt, das von den Karotiden des Halses zum Kopf fließt,
**dadurch gekennzeichnet, dass** die obere Auflage ferner ein erstes längliches Halselement (56) an der rechten Seite und ein zweites längliches Halselement (58) an der linken Seite aufweist, wobei die Halselemente im Wesentlichen über den Karotiden des Halses angeordnet sind, wenn sie von dem Individuum getragen werden.

2. Kühlsystem wie in Anspruch 1 dargelegt, wobei die oberen und hinteren Auflagen ein flexibles Material umfassen, dass sich an den Kopf und Hals eines Individuums anpasst.

3. Kühlsystem wie in Anspruch 1 dargelegt, wobei die länglichen Halselemente jeweils ferner eine Umfangskontur umfassen, die geformt ist, um Zugang zu den Ohren des Individuums bereitzustellen.

4. Kühlsystem wie in Anspruch 1 dargelegt, das ferner einen Halsgurt (32) umfasst, wobei die obere Auflage, die ein oder mehrere Befestigungselemente aufweist, beweglich am Halsgurt befestigbar ist.

5. Kühlsystem wie in Anspruch 4 dargelegt, wobei die hintere Auflage ferner ein Befestigungselement umfasst, wobei die hintere Auflage beweglich am Halsgurt befestigbar ist.

6. Kühlsystem wie in Anspruch 1 angegeben, wobei das System ein drittes Element umfasst, das aktivierbares Kühlmittel beherbergt, wobei das dritte Element beweglich an der hinteren Auflage befestigt ist, um bei Aktivierung des Kühlmittels Kühlung der Spina bereitzustellen.

7. Kühlsystem wie in Anspruch 4 angegeben, wobei der Halsgurt ferner ein flexibles, festes Material zur Stützung des Halses umfasst.

8. Kühlsystem wie in Anspruch 4 angegeben, wobei der Halsgurt ferner eine oder mehrere Öffnungen (48) zur Belüftung und Atmungsunterstützung umfasst.

9. Kühlsystem wie in Anspruch 4 angegeben, wobei der Halsgurt ferner ein vorderes Gurtelement (44) und ein hinteres Gurtelement (46) umfasst.

10. Kühlsystem wie in Anspruch 4 angegeben, wobei der Halsgurt ferner definiert ist als mindestens eine Öffnung umfassend, die den Zugang zum Hals des Individuums erlaubt.

## Revendications

1. Système de refroidissement pour refroidir la tête et le cou d'un individu portant le système, le système comportant :
un panneau supérieur (34) ayant une ou plusieurs attaches coopérantes et ayant un côté droit et un côté gauche correspondant au côté droit et au côté gauche, respectivement, dudit individu ;
un panneau arrière (36) ayant une ou plusieurs attaches coopérantes, par lesquelles ledit panneau supérieur peut être attaché de manière ajustable sur ledit panneau arrière ; et
un milieu de refroidissement activable (98) logé dans ledit panneau supérieur et dans ledit panneau arrière, ledit milieu de refroidissement refroidissant la tête et refroidissant du sang s'écoulant vers la tête depuis les artères carotides du cou lors de l'activation dudit milieu de refroidissement ;
**caractérisé en ce que** ledit panneau supérieur a en outre un premier élément de cou allongé (56) dudit côté droit, et un second élément de cou allongé (58) dudit côté gauche, de sorte que lesdits éléments de cou sont disposés sensiblement au dessus des artères carotides du cou lorsqu'ils sont portés par ledit individu.

2. Système de refroidissement selon la revendication 1, dans lequel lesdits panneaux supérieur et arrière comportent un matériau flexible qui se conforme à la tête et au cou d'un individu.

3. Système de refroidissement selon la revendication 1, dans lequel lesdits éléments de cou allongés comportent chacun en outre un contour périphérique mis en forme pour fournir un accès aux oreilles dudit individu.

4. Système de refroidissement selon la revendication 1, comportant en outre un collier (32), dans lequel ledit panneau supérieur ayant une ou plusieurs attaches peut être attaché de manière amovible audit collier.

5. Système de refroidissement selon la revendication 4, dans lequel ledit panneau arrière comporte en outre une attache, par laquelle ledit panneau arrière peut être attaché de manière amovible sur ledit collier.

6. Système de refroidissement selon la revendication 1, dans lequel ledit système comporte un troisième élément logeant un milieu de refroidissement activable de sorte que le troisième élément est attaché de manière amovible audit panneau arrière pour fournir un refroidissement de la colonne vertébrale lors de l'activation dudit milieu de refroidissement.

7. Système de refroidissement selon la revendication 4, dans lequel ledit collier comporte en outre un matériau rigide en flexion pour supporter le cou.

8. Système de refroidissement selon la revendication 4, dans lequel ledit collier comporte en outre une ou plusieurs ouvertures (48) pour la ventilation d'air et l'assistance respiratoire.

9. Système de refroidissement selon la revendication 4, dans lequel ledit collier comporte en outre un élément de collier avant (44) et un élément de collier arrière (46).

10. Système de refroidissement selon la revendication 4, dans lequel ledit collier est en outre défini comme comportant au moins un orifice permettant l'accès au cou dudit individu.
